(19) Europäisches Patentamt

European Patent Office

Office européen des brevets

(11) Veröffentlichungsnummer: **0 280 844 B1**

(12) **EUROPÄISCHE PATENTSCHRIFT**

(45) Veröffentlichungstag der Patentschrift: **03.06.92**

(51) Int. Cl.⁵: **A61K 7/50**, C11D 10/04

(21) Anmeldenummer: **88100246.3**

(22) Anmeldetag: **11.01.88**

(54) **Körperpflegemittel auf Seifenbasis.**

(30) Priorität: **04.02.87 DE 3703258**

(43) Veröffentlichungstag der Anmeldung:
**07.09.88 Patentblatt 88/36**

(45) Bekanntmachung des Hinweises auf die
Patenterteilung:
**03.06.92 Patentblatt 92/23**

(84) Benannte Vertragsstaaten:
**AT BE CH DE ES FR GB GR IT LI LU NL SE**

(56) Entgegenhaltungen:
**DE-A- 1 767 143**
**FR-A- 2 338 326**
**US-A- 3 988 438**
**US-A- 4 285 826**

(73) Patentinhaber: **Singh-Verma, Shyam Bir, Dr.**
**Nussbaumallee 13**
**W-5014 Kerpen(DE)**

(72) Erfinder: **Singh-Verma, Shyam Bir, Dr.**
**Nussbaumallee 13**
**W-5014 Kerpen-Türnich(DE)**
Erfinder: **Jordan, Josef**
**Aus Hauserbach 28**
**W-5014 Kerpen(DE)**
Erfinder: **Sossna, Raimund**
**Geilenkirchener Strasse 1**
**W-5000 Köln 41(DE)**

(74) Vertreter: **Naumann, Ulrich et al**
**Ullrich & Naumann, Patentanwälte, Gaisberg-**
**strasse 3**
**W-6900 Heidelberg 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 280 844 B1

**Beschreibung**

Die Erfindung betrifft ein Körperpflegemittel auf der Basis von Alkalisalzen wenigstens einer vorgegebenen Fettsäure (Seife) als waschaktiver Substanz mit Zusätzen von Farben und/oder Geruchsstoffen und/oder hautpflegenden Substanzen und/oder Konditionierungsmitteln.

Beim Reinigen der Haut mit einem Körperpflegemittel auf Seifenbasis, d.h. auf der Basis von Alkalisalzen bestimmter Fettsäuren wird die Haut von Hauttalgstauungen, Schmutz und Resten vorher aufgebrachter kosmetischen Mittel wie Puder, Cremeresten oder dergleichen befreit. Eine herkömmliche Seife hat insbesondere bei empfindlicher Haut erhebliche Nachteile in bezug auf die Hautphysiologie. So wird durch Seifen der natürliche Hydrolipid-Mantel der Haut angegriffen. Der Säureschutzmantel der Haut wird durch das Alkali der Seife zerstört, so daß selbst bei intensivem Nachspülen mit Wasser der natürliche Zustand erst in etwa 1 - 1,5 Stunden wieder hergestellt ist. Das Waschen mit Seife fuhrt außerdem zu einer erheblichen Quellung der Haut, die bei gesunder Haut zwar von untergeordneter Bedeutung ist. Bei angegriffener, empfindlicher oder kranker Haut, wie beispielsweise bei Ekzemen, Akne oder dergleichen führt dies jedoch zum Austrocknen oder zur Rißbildung, also zu sogenannter "rauher" Haut.

Um diese Nachteile zu vermeiden, hat man auch synthetische waschaktive Substanzen, sogenannte Syndets, als Körperpflegemittel eingesetzt. Diese haben ein gutes Schaumvermögen, eine höhere Reinigungskraft. Da eine Quellung der Haut hierbei vermieden wird, sind zur Reinigung einer angegriffenen, empfindlichen oder erkrankten Haut diese Syndets geeignet. Diesen Vorteilen gegenüber einer herkömmlichen Seife stehen Nachteile gegenüber: so wird die Haut stark entfettet, was wiederum zur Austrocknung und Rißbildung führen kann. Ferner wird die natürliche Hautflora erheblich beeinträchtigt. Daher müssen Syndets in der Regel rückfettende Substanzen zugefügt werden.

Aus US-A-4 285 826 ist für eine Toilettenseife bekannt, zur Verbesserung des Feuchtigkeitshaushaltes der Haut und zur Verbesserung des Hautgefühls einer natürlichen Seifenmischung ethoxylierte Alkohole zuzugeben, die den gewünschten Effekt durch eine Überfettung der Seife erreicht.

Aus DE-A-17 67 143 ist ein reines Textilwaschmittel zur Verwendung in Trommelwaschmaschinen auf der Basis von Syndets bekannt, dem Seife als Schaumregulanz beigefügt ist. Die in dieser Veröffentlichung angegebenen Syndets sind als hautschädigend zu beurteilen. Eine Anregung zur Formulierung eines Körperpflegemittels war für einen Fachmann hieraus nicht abzuleiten.

Der Erfindung liegt nun die Aufgabe zugrunde, ein Körperpflegemittel auf Seifenbasis, d.h. auf der Basis von Alkalisalzen bestimmter Fettsäuren zu schaffen, das die Vorteile einer Seife und die Vorteile einer synthetischen waschaktiven Substanz miteinander vereinigt, so daß eine wesentlich verbesserte hautphysiologische Wirkung erzielt wird.

Diese Aufgabe wird dadurch gelöst, daß als weitere waschaktive Substanz wenigstens ein synthetisches Detergens (Syndet) in Form eines Carboxymethylethers von ethoxylierten Fettsäuren und/oder Fettsäurederivaten mit der Seife vermischt ist und daß das Mischungsverhältnis zwischen Seife und Syndet in Gewichtsprozenten im Bereich von 92 : 3 bis 65 : 30 liegt.

Der verbleibende Rest zu 100 % wird durch die üblichen Zusätze zu Körperpflegemitteln, wie im Oberbegriff des Anspruchs 1 angegeben, ausgefüllt. Eine derartige Mischung einer Seife mit der angegebenen Syndetklasse hat den Vorteil einer gegenseitigen positiven Beeinflussung der Komponenten und zwar sowohl hinsichtlich der hautphysiologischen Eigenschaften als auch hinsichtlich der Verarbeitbarkeit. Die Seifen und die angegebene Syndetklasse läßt sich zu einer homogenen Masse mischen, die sich bei flüssiger oder pastöser Darreichungsform nicht trennt und die für die stückige Darreichungsform einwandfrei preßbar ist. Bevorzugt ist hierbei ein Miverhältnis im Bereich zwischen 90 : 5 bis 70 : 25, insbesondere im Bereich 85 : 10 bis 75 : 20 vorgesehen.

In einer Ausgestaltung der Erfindung ist für eine fließfähige Darreichungsform, und zwar für eine flüssige Konsistenz ein Gesamtgehalt an waschaktiven Substanzen von 15 bis 40 Gewichtsprozent und für eine pastöse Konsistenz ein Gesamtgehalt an waschaktiven Substanzen von 30 bis 80 Gewichtsprozent bezogen auf das Gesamtgewicht vorgesehen.

In einer anderen Gestaltung ist für eine stückförmige Darreichungsform der Gesamtgehalt an waschaktiven Substanzen von mindestens 80 Gewichtsprozent bezogen auf das Gesamtgewicht vorgesehen.

Bevorzugte Ausgestaltungen der Erfindung sind in den Unteransprüchen 6 bis 9 angegeben. So können als Syndet die Alkalisalze wenigstens eines Carboxymethylethers bestimmter ethoxylierter Fettsäureamide oder Fettsäuren mit der Seife kombiniert werden. Die Mischung führt zu einem Körperpflegemittel, das in hervorragender Weise die Vorteile der beiden Komponenten in sich vereinigt. Es zeichnet sich durch einen besonders cremigen und stabilen Schaum sowie durch ein besonders angenehmes Hautgefühl sowohl beim Waschvorgang selbst wie auch nach dem Abspülen und Abtrocknen aus. Die Haut wird weniger beansprucht, da die Quellung und die Entfettung der Haut in starkem Maße reduziert wird. Insgesamt ergibt sich nach der Anwendung ein angenehmes Hautgefühl, das in bisher nicht bekannter Weise als ausgesprochen mild und hautsympathisch zu bezeichnen ist. Es hat sich ferner gezeigt, daß insbesondere gegenüber herkömmlichen, reinen Seifenstücken die Parfümierung klar zum Ausdruck kommt, so daß die Menge der zuzufügenden Geruchsstoffe erheblich reduziert werden kann. Dabei bleibt bei einem stückförmigen Körperpflegemittel der erfindungsgemäßen Art die Duftwirkung nach der Anwendung lange erhalten. Außerdem wird das Reißen von stückförmigen Darreichungsformen völlig unterbunden. Auch die Bildung von Kalkseifen, die häßliche Ränder an Waschbecken oder Badewannen verursachen, wird verhindert oder stark zurückgedrängt.

In einer anderen Ausgestaltung der Erfindung ist vorgesehen, daß anstelle des Alkalisalzes wenigstens eines Carboxymethylethers bestimmter ethoxylierter Fettsäureamide oder Fettsäuren die Mono-, Di- oder Triethanolaminsalze eingesetzt werden. Die eingesetzten Syndets weisen zweckmässigerweise einen Ethoxylierungsgrad zwischen 2 und 20 auf. Als Fettsäuren bzw. Fettsäureamid kommen in allen vorgenannten Ausgestaltungen der Erfindung insbesondere die Laurinsäure, die Myristinsäure, die Palmitinsäure, die Stearinsäure und die Ölsäure bzw. ihre Amide sowie Mischungen derselben in Betracht.

Bevorzugt wird die Mischung von nur einer Syndetklasse mit der Seife, da dann die gegenseitige positive Beeinflussung der Komponenten am günstigsten ist. Die Verwendung von zwei oder mehreren unterschiedlichen Syndets kann dazu führen, daß diese sich sowohl in bezug auf die hautphysiologische Wirkung als auch hinsichtlich der Verarbeitbarkeit negativ beeinflussen. Der besondere Vorteil der angegebenen Syndetklassen liegt insbesondere darin, daß sich die Wirkung der Seifen einerseits und der Syndets andererseits positiv beeinflussen, was zu den vorstehend dargelegten Vorteilen führt.

Versucht man jedoch, Seifen mit herkömmlichen Syndets wie Benzylsulfonaten, Fettsäureethersulfaten, Fettsäuresulfosuccinaten oder chemisch ähnlich aufgebauten waschaktiven Substanzen zu mischen, so lassen sich diese Gemische aufgrund ihrer Konsistenz nicht zu kosmetisch befriedigenden Darreichungsformen verarbeiten. Darüber hinaus weisen diese auch nicht die gewünschten guten hautphysiologischen Effekte auf.

**Patentansprüche**

1. Körperpflegemittel auf der Basis von Alkalisalzen wenigstens einer vorgegebenen Fettsäure (Seife) als waschaktive Substanz mit Zusätzen von Farben und/oder Duftstoffen und/oder hautpflegenden Substanzen und/oder Konditionierungsmitteln, dadurch **gekennzeichnet,** daß als weitere waschaktive Substanz wenigstens ein synthetisches Detergens (Syndet) in Form eines Carboxymethylethers von ethoxylierten Fettsäuren und/oder Fettsäurederivaten mit der Seife vermischt ist und daß das Mischungsverhältnis zwischen Seife und Syndet in Gewichtsprozent im Bereich von 92 : 3 bis 65 : 30 liegt.

2. Körperpflegemittel nach Anspruch 1, dadurch gekennzeichnet, daß das Mischungsverhältnis zwischen Seife und Syndet in Gewichtsprozent im Bereich von 90 : 5 bis 70 : 25, vorzugsweise im Bereich von 85 : 10 bis 75 : 20 liegt.

3. Körperpflegemittel nach Anspruch 1 oder 2, dadurch gekennzeichnet, daß für eine fließfähige Darreichungsform der Gesamtgehalt an waschaktiven Substanzen 15 bis 40 Gewichtsprozent, bezogen auf das Gesamtgewicht, beträgt.

4. Körperpflegemittel nach Anspruch 1 oder 2, dadurch gekennzeichnet, daß für eine pastöse Darreichungsform der Gesamtgehalt an waschaktiven Substanzen zwischen 30 und 80 Gewichtsprozent, bezogen auf das Gesamtgewicht beträgt.

5. Körperpflegemittel nach Anspruch 1 oder 2, dadurch gekennzeichnet, daß für eine stückförmige Darreichungsform der Gesamtgehalt an waschaktiven Substanzen mindestens 80 Gewichtsprozent, bezogen auf das Gesamtgewicht beträgt.

6. Körperpflegemittel nach einem der Ansprüche 1 bis 5, dadurch gekennzeichnet, daß als Syndet Carboxymethylether von ethoxylierten Fettsäuren oder deren Alkalisalzen, vorzugsweise deren Natriumsalzen eingesetzt werden.

7. Körperpflegemittel nach einem der Ansprüche 1 bis 5, dadurch gekennzeichnet, daß als Syndet Carboxymethylether von ethoxylierten Fettsäuren oder deren Ammoniumsalzen, insbesondere Mono-, Di- oder Triethanolaminsalzen eingesetzt werden.

8. Körperpflegemittel nach einem der Ansprüche 1 bis 5, dadurch gekennzeichnet, daß als Syndet Carboxymethylether von ethoxylierten Fettsäureamiden oder deren Alkalisalzen, vorzugsweise deren Natriumsalzen eingesetzt werden.

9. Körperpflegemittel nach einem der Ansprüche 1 bis 5, dadurch gekennzeichnet, daß als Syndet Carboxymethylether von ethoxylierten Fettsäureamiden oder deren Ammoniumsalzen, insbesondere Mono-, Di- oder Triethanolaminsalzen eingesetzt werden.

10. Körperpflegemittel nach einem der Ansprüch 6 bis 9, dadurch gekennzeichnet, daß der Ethoxylierungsgrad des eingesetzten Syndets zwischen 2 und 20 liegt.

11. Körperpflegemittel nach einem der Ansprüche 1 bis 10, dadurch gekennzeichnet, daß vorwiegend die Laurinsäure, Myristinsäure, Palmitinsäure, Stearinsäure oder Ölsäure oder deren Mischungen als zugrundeliegende Fettsäuren in Syndets vorgegeben werden.

## Claims

1. Body care agent based on alkali salts of at least one predetermined fatty acid (soap) as a substance with a washing effect with the addition of colorants and/or perfume substances and/or substances which care for the skin and/or conditioning agents, characterised in that at least one synthetic detergent (syndet) in the form of a carboxymethyl ether of oxyethylated fatty acids and/or fatty acid derivatives is mixed with the soap as a further substance with a washing effect; and in that the mixture ratio of soap to syndet ranges from 92 : 3 to 65 : 30 weight percent.

2. Body care agent according to Claim 1, characterised in that the mixture ratio of soap to syndet ranges from 90 : 5 to 70 : 25, and preferably from 85 : 10 to 75 : 20 weight percent.

3. Body care agent according to Claim 1 or 2, characterised in that in order to be available in a flowable form the total content of substances with a washing effect is 15 to 40 weight percent relative to the total weight.

4. Body care agent according to Claim 1 or 2, characterised in that in order to be available in paste form the total content of substances with a washing effect is between 30 and 80 weight percent relative to the total weight.

5. Body care agent according to Claim 1 or 2, characterised in that in order to be available in pieces the total weight of substances with a washing effect is at least 80 weight percent relative to the total weight.

6. Body care agent according to any one of Claims 1 to 5, characterised in that carboxymethyl ether of oxyethylated fatty acids or their alkali salts, preferably their sodium salts, are used as syndet.

7. Body care agent according to any one of Claims 1 to 5, characterised in that carboxymethyl ether of oxyethylated fatty acids or their ammonium salts, in particular mono-, di- or triethanol amine salts, are used as syndet.

8. Body care agent according to any one of Claims 1 to 5, characterised in that carboxymethyl ether of oxyethylated fatty acid amides or their alkali salts, preferably their sodium salts, are used as syndet.

9. Body care agent according to any one of Claims 1 to 5, characterised in that carboxymethyl ether of oxyethylated fatty acid amides or their ammonium salts, in particular mono-, di- or triethanol amine salts, are used as syndet.

10. Body care agent according to any one of Claims 6 to 9, characterised in that the degree of oxyethylation of the syndet used is between 2 and 20.

11. Body care agent according to any one of Claims 1 to 10, characterised in that lauric acid, myristic acid, palmitic acid, stearic acid or oleic acid or their mixtures are predominantly used as the basic fatty acids in the syndet.

## Revendications

1. Produit cosmétique à base de sels alcalins d'au moins un acide gras prédéterminé (savon) en tant que substance active pour le lavage et comportant des additifs de colorants et/ou des substances odoriférantes et/ou des substances pour les soins de la peau et/ou des agents de conditionnement, caractérisé en ce qu'en tant qu'autre substance active pour le lavage, au moins un détersif synthétique (détergent synthétique) sous la forme d'un carboxyméthyléther d'acides gras éthoxylés et/ou de dérivés d'acides gras est mélangé au savon, et que le rapport de mélange entre le savon et le détersif synthétique est compris dans la gamme allant de 92:3 à 65:30, en pourcentages en poids.

2. Produit cosmétique selon la revendication 1, caractérisé en ce que le rapport de mélange entre le savon et le détersif synthétique est compris dans la gamme allant de 90:5 à 70:25 et de préférence dans la gamme allant de 85:10 à 75:20, en pourcentage en poids.

3. Produit cosmétique selon la revendication 1 ou 2, caractérisé en ce que pour une forme de présentation à l'état fluide, la teneur totale en substances actives pour le lavage est comprise entre 15 et 40 pour cent en poids rapportés au poids total.

4. Produit cosmétique selon la revendication 1 ou 2, caractérisé en ce que pour une forme de présentation à l'état de pâte, la teneur totale en substance active pour le lavage est comprise entre 30 et 80 pour cent en poids, rapportés au poids total.

5. Produit cosmétique selon la revendication 1 ou 2, caractérisé en ce que pour une forme de représentation à l'état de blocs, la teneur totale en substance active pour le lavage est égale au moins à 80 pour cent en poids rapportés au poids total.

6. Produit cosmétique selon l'une des revendications 1 à 5, caractérisé en ce qu'on utilise comme détersif synthétique, des carboxyméthyléthers d'acides gras éthoxylés ou leurs sels alcalins, de préférence leurs sels de sodium.

7. Produit cosmétique selon l'une des revendications 1 à 5, caractérisé en ce qu'on utilise comme détersif synthétique, des carboxylméthyléthers d'acides gras éthoxylés ou leurs sels d'ammonium, notamment leurs sels de monoéthanolamine, diéthanolamine ou triéthanolamine.

8. Produit cosmétique selon l'une des revendications 1 à 5, caractérisé en ce qu'on utilise comme détersif synthétique, des carboxyméthyléthers d'amides éthoxylés d'acides gras ou leurs sels alcalins, de préférence de leurs sels de sodium.

9. Produit cosmétique selon l'une des revendications 1 à 5, caractérisé en ce qu'on utilise, comme détersif synthétique, des carboxyméthyléthers d'amides éthoxylés d'acides gras ou leurs sels d'ammonium, notamment leurs sels de monoéthanolamine, diéthanolamine ou triéthanolamine.

10. Produit cosmétique selon l'une des revendications 6 à 9, caractérisé en ce que le degré d'éthoxylation du détersif synthétique utilisé est compris entre 2 et 20.

11. Produit cosmétique selon l'une des revendications 1 à 10, caractérisé en ce qu'on choisit de préférence, en tant qu'acides gras de base dans des détersifs synthétiques, l'acide laurique, l'acide myristique, l'acide palmitique, l'acide stéarique ou l'acide oléique ou leurs mélanges.